# EUROPEAN PATENT APPLICATION

(11) **EP 3 156 049 A1**
(43) Date of publication of application: **19.04.2017**
(21) Application number: 16194014.3
(22) Date of filing: 14.10.2016
(51) Int. Cl.: A61K 9/20, A61K 31/4365

(54) **PHARMACEUTICAL COMPOSITION OF PRASUGREL**

(30) Priority: 15.10.2015 IN 3906MU2015
(71) Applicant: Alembic Pharmaceuticals Limited, Vadodara 390 003 (IN)
(72) Inventor: TADURI, Veerababu Ramabrahmmam, 390 003 Gujarat (IN); GIRISH, Achliya, 390 003 Gujarat (IN); SUNDERRAJ, Manvi, 390 003 Gujarat (IN); SAGAR, Seth, 390 003 Gujarat (IN); GAURAV, Mehta, 390 003 Gujarat (IN)
(74) Representative: Srinivasan, Ravi Chandran

(57) **Abstract**

The present invention relates to a stabilized pharmaceutical composition comprising Prasugrel base in micronized form, a surfactant and / or a stabilizer which is bioequivalent to the EFIENT^{®} (prasugrel hydrochloride marketed formulation). The present invention further relates to process for the preparation of stabilized pharmaceutical compositions.

## Description

### FIELD OF THE INVENTION

The present invention relates to a stabilized pharmaceutical composition comprising Prasugrel base in micronized form, a surfactant and / or a stabilizer. The present invention further relates to process for the preparation of stabilized pharmaceutical compositions.

### BACK GROUND OF THE INVENTION

Prasugrel is a thienopyridine class inhibitor of platelet activation and aggregation through the irreversible binding of its active metabolite to the P2Y₁₂ class of ADP receptors on platelets.

A product containing Prasugrel Hydrochloride was approved for marketing in US with the Brand name EFFIENT^{®} as 5 mg and 10 mg film coated tablets. EFFIENT^{®} is formulated with a hydrochloride salt of Prasugrel which is chemically designated as 5-[(1RS)-2-cyclopropyl-1-(2-fluorophenyl)-2-oxoethyl]-4, 5, 6, 7-tetrahydrothieno [3, 2-c] pyridin-2-yl acetate hydrochloride. Prasugrel hydrochloride has the empirical formula C₂₀H₂₀FNO₃S•HCl representing a molecular weight of 409.90. The chemical structure of Prasugrel hydrochloride is

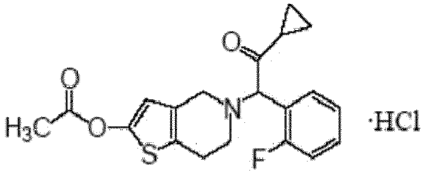

U.S.Patent No 5,288,726 discloses tetrahydrothiene derivatives including prasugrel. The said document discloses the use of these compounds for treatment and prophylaxis of thrombosis and embolisms.

U.S. Patent No. 6,693,115 discloses the hydrochloric acid and maleic acid salts of Prasugrel.

EP 2564847 Discloses prasugrel composition for improving solubility, dissolution and bioavailability. However, improving bioavailability is not mean that product is bioequivalent. It is necessary to develop formulation that is safe and bioequivalent to that of Efient^{®}

Prasugrel hydrochloride is a white solid, which is soluble at pH 2, slightly soluble at pH 3 to 4, and practically insoluble at pH 6 to 7.5.

Prasugrel hydrochloride is susceptible to hydrolysis and therefore contact with water should be avoided. As it is susceptible to both hydrolytic and oxidative degradation approaches require to maintain stability of product during formulation, manufacturing process and packaging of the dosage form. Earlier it is demonstrated that hydrochloride salt was more susceptible to hydrolysis then the free base but it has less solubility as compare to hydrochloride salt.

Hence, there is a long felt need to prepare a solid dosage form of prasugrel base which has improved solubility, stability and is bioequivalent to the EFIENT^{®} (prasugrel hydrochloride formulation).

It has been found that using Prasugrel base in micronized form with a surfactant and /or a stabilizer gives pharmaceutical formulation with improved solubility, stability and is bioequivalent to EFIENT^{®}.

### SUMMARY OF THE INVENTION

In one general aspect, there is provided a stabilized pharmaceutical composition comprising Prasugrel base in micronized form,surfactant and/or a stabilizer.

In another aspect of invention stable oral pharmaceutical composition comprising Prasugrel base, at least one surfactant having stabilizing effect and one or more pharmaceutically acceptable excipients.

In yet another aspect there is provided a process for the preparation of stabilized pharmaceutical composition comprising the steps of:
a) Dissolving a Prasugrel and surfactant in the acetone;
b) Dissolving binder in Acetone: Methanol mixture;
c) Mixing of the mixtures of step a) and b);
d) Granulating the mixture of step c) with inactive excipients including optionally stabilizers by top spray granulation technology followed by drying;
e) Mixing the dried granules with one or more pharmaceutically acceptable excipients;
f) Compressing the blend to form the tablet and
g) Optionally coating the tablet.

### DETAILED DESCRIPTION OF THE INVENTION

The present invention relates to stabilized pharmaceutical compositions of Prasugrel comprising Prasugrel base in micronized form and surfactant and /or a stabilizer.

The present invention further relates to stable oral pharmaceutical composition comprising Prasugrel base, at least one surfactant having stabilizing effect and one or more pharmaceutically acceptable excipients.

The stabilized pharmaceutical composition, like those that described herein, may comprise the Prasugrel base in micronized form, a surfactant which is poloxamer and/ or Docusate sodium and /or a stabilizer which is cyclodextrin and optionally one or more pharmaceutically acceptable excipients.

The term "Prasugrel base in micronized form" as used here in means a particle size of prasugrel base containing particles prepared by any process or methods of particle size reduction. For example particle size reduction of prasugrel base of present invention can be obtained by any milling, grinding, micronizing or any particle size reduction method known in the art. Further the micronized prasugrel base having particle as per following.
D10 is not more than 5.0 microns
D50 is Between 5.0 to 10.0 microns
D90 is not more than 20.0 microns.

A surfactant is an amphiphilic material having hydrophilicity and lipophilicity. The surfactant can improve the drug wettability; avoid drug particle coalescence, with the help of the micellar solubilization, which helps to improve solubility as well as bioavailability for poorly soluble drug. Types of surfactant can be divided into four categories: anionic surfactant, cationic surfactant, amphiphilic surfactant and non-ionic surfactant.

A stabilizer is a material which helps to stabilize the unstable molecules which include cyclodextrin and like.

The stabilized pharmaceutical compositions of the present invention may be prepared by any known processes of manufacturing the pharmaceutical formulation to the person having ordinary skill in the art of pharmaceutical technology such as solid dispersion,direct compression, wet granulation, dry granulation, slugging, hot melt extrusion, hot melt granulation, extrusion-spheronization, fluidized bed granulation, spray drying and solvent evaporation.

In one embodiment the stabilized pharmaceutical composition of the present invention can be prepared by solid dispersion using top spray granulation of Prasugrel base in micronized form, a surfactant which is poloxamer and/ or docusate sodium and optionally a stabilizer which is cyclodextrin and optionally mixing with one or more pharmaceutically acceptable excipients. More preferably solid dispersion method was used to formulate stabilized pharmaceutical composition of prasugrel base. The preferred solvent is selected from but not limited to acetone, methanol, ethanol, propanol , methylene chloride, ethyl acetate, chloroform, DMF or/and ethyl ether and its combination thereof and like. Preferably selected from acetone or/and methanol.

### Stabilized pharmaceutical composition

The above described Prasugrel base in micronized form, a surfactant which is poloxamer and/ or docusate sodium and/or a stabilizer which is cyclodextrin can subsequently processed by using pharmaceutically acceptable excipients to form stabilized pharmaceutical composition which can be used in particular for the reduction of Thrombotic cardiovascular events.

The finished stabilized pharmaceutical composition can be a powder, fine granules, granules, a capsule or a tablet and preferably it is a tablet.

In addition to poloxamer and/ or docusate sodium which act as surfactant and cyclodextrin which act as a stabilizer, the stabilized pharmaceutical composition can further contain one or more pharmaceutically acceptable excipients, such as fillers, binders, lubricants, disintegrants and coating agents.

### Surfactants

The function of surfactant is to improve the drug wettability, with the help of the micellar solubilization, which improves the solubility and thereby bioavailability of drug. Surface-active agent, including substances commonly referred to as wetting agents, surface tension depressants, detergents, dispersing agents, emulsifiers, and quaternary ammonium antiseptics. Preferred examples of surfactants are but not limited to Poloxamer, Docusate sodium, Sodium lauryl sulphate, polysorbate etc.

Preferably the surfactant is an anionic surfactant or a non-ionic surfactant.

Preferably the anionic surfactant is a sulfonate, e.g. an organic sulfonate, more preferably a diester substituted by sulfonate, and yet more preferably a 1,4-bis(alkoxy)-1,4-dioxobutanesulfonate. In this regard each alkoxy group is preferably a C₄-C₂₀alkoxy group, more preferably a C₆-C₁₀alkoxy group, and most preferably a C₈alkoxy group such as 2-ethylhexoxy. Thus, a preferred option for the anionic surfactant is a docusate salt, with docusate sodium being most preferred.

The anionic surfactant is preferably in the form of a pharmaceutically acceptable salt. Preferably the anionic surfactant is a salt of an alkali metal or an alkaline earth metal, more preferably an alkali metal such as sodium.

Preferably the non-ionic surfactant is a copolymer, typically a block copolymer, e.g. a triblock copolymer. Preferably the triblock copolymer comprises a central hydrophobic chain flanked by two hydrophilic chains. The hydrophobic chain is preferably a polyoxypropylene. The hydrophilic chains are preferably polyoxyethylene. Thus, a preferred option for the copolymer is a poloxamer.

The copolymer non-ionic surfactant may have a number average molecular weight of e.g. 1,000 to 100,000, such as 3,000 to 50,000 or 5,000 to 20,000.

### Stabilizers

A stabilizer is a material which helps to stabilize the unstable molecules which include cyclodextrin and like by the method of mixing of active and other excipients, inclusion complex preferably by mixing with the active and other excipients.

### Fillers

A stabilized pharmaceutical composition described herein can further contain one or more fillers. In general, filler is a substance increasing the bulk volume of mixture and thus the size of final dosage form. Preferred examples of fillers are microcrystalline cellulose, powdered cellulose, lactose (anhydrous or monohydrate), starch (e.g., corn starch or potato starch), pregelatinized starch, sugar and sugar alcohol like mannitol, fructose, sucrose, dextrose, dextrans, sorbitol , saccharose; , calcium hydrogen phosphate, calcium hydrogen phosphate dihydrate, dicalciumphosphatedihydrate, tricalciumphophate, or mixtures thereof. The preferred filler is microcrystalline cellulose and mannitol.

### Binders

The stabilized pharmaceutical compositions described herein can further contain binders. In general, binder is the substance which is used to bind other material together in such a way that it maintains a uniform consistency of the mixture. Preferred examples of binders are hydroxypropylcellulose (HPC), hydroxypropylmethylcellulose (HPMC), hydroxyethylcellulose (HEC), starch (e.g., corn starch or potato starch), pregelatinized starch, silicified microcrystalline cellulose, povidone, cellulose derivatives (e.g., methylcellulose and sodium carboxymethylcellulose), gelatin, polyethylene glycol and sodium alginate. The preferred binder is hydroxypropylcellulose.

### Disintegrants

The function of the disintegrants is to break up the tablet in to smaller pieces as soon as it contact with a liquid. Preferred examples of disintegrants are cross carmellose sodium (CCS), cross linked polyvinyl pyrollidone (Crosprovidone), sodium starch glycolate (SSG) and sodium carboxy methyl starch. The preferred disintegrant is cross carmellose sodium.

### Lubricants

Lubricants ensure that tablet formation and ejection can occur with low friction between the dosage forms (solid) and die wall. Preferred examples of lubricants are Sodium Stearyl Fumarate (SSF), Sucrose stearate, magnesium stearate, stearic acid, calcium stearate, hydrogenated vegetable oil, hydrogenated castor oil, macrogols, glycerylbehenate, talc, silicone dioxide or mixtures thereof. The preferred lubricant is magnesium stearate, sodium stearyl fumarate and / or sucrose stearate.

### Coating agents

Optionally, pharmaceutical compositions described herein, including cores, can be coated with conventional materials used for film coating or rate controlling polymers for functional coating. Film coating compositions usually contain the following components: polymer(s), plasticizer(s), colorant(s)/opacifier(s), vehicle(s). Minor quantities of flavours, surfactants and waxes also can be used in the film coating solution or suspension. The majority of the polymers used in film coatings are cellulose derivatives and others, such as the hydroxypropylmethylcellulose, hydroxypropylcellulose, polyvinylpyrrolidone and polyvinyl alcohol.

The plasticizers that is to be used for the preparation of film coating solution/dispersions can be selected from the group consisting of glycerol, propylene glycol, macrogols, phthalate esters, dibutylsebacetate, citrate esters, triacetin, castor oil and acetylated monoglycerides.

Film coats may be prepared from ready-to-make preparations which are available in the market. One preferred film-coat material is OPADRY II Brown. Depending on the desired opacity, the preferred amount of the coating is about 2-5% w/w of the tablet.

A film coating dispersion or suspension can be prepared by using different solvents (water, alcohols, ketones, esters, chlorinated hydrocarbons) preferably water is to be used as solvent.

One more embodiment of the present invention is to provide a method of treating patients to reduce the rate of thrombotic cardiovascular events with acute coronary syndrome by administering a stabilized pharmaceutical composition of the present invention. In particular, disclosed is a method of treating patients suffering from unstable angina, Non-ST-Elevation Myocardial Infraction (NSTEMI) and ST-Elevation Myocardial Infraction (STEMI) by administering a unit dosage form of the stabilized pharmaceutical composition comprising Prasugrel base as described herein.

Further embodiment of the present invention describes the process for the preparation of the stabilized pharmaceutical composition for oral administration comprising
a) Dissolving a Prasugrel base and surfactant in the acetone;
b) Dissolving binder in Acetone: Methanol mixture;
c) Mixing of the mixtures of step a) and b);
d) Granulating the mixture of step c) with inactive excipients including stabilizers by top spray granulation technology followed by drying;
e) Mixing the dried granules with one or more pharmaceutically acceptable excipients;
f) Compressing the blend to form the tablet and
g) Optionally coating the tablet.

Process for preparation of formulation by solid dispersion enhance the solubility of prasugrel base and thereby improve the dissolution. Further formulation prepared by solid dispersion process having less intra-subject variability as compare to wet granulation process.

### Packaging:

As prasugrel base is susceptible to both hydrolytic and oxidative degradation, the formulation, manufacturing process, and packaging is also important for maintaining the stability of the product.

According to present invention, Packaging are of different types and it can be selected from, but not limited to Alu-Alu blister with desiccant tablet, Alu-Alu Blister, Alu-Alu blister with desiccant layer and HDPE bottles.

In one embodiment the present formulation is stable up to 6 month in Alu-Alu blister with desiccant tablet and Alu-Alu Blister at 40°C/75% RH.

The preparation of the stabilized pharmaceutical composition of Prasugrel base will be described in more detail by the way of examples. The following examples will serve to illustrate the practice of this invention, it is being understood that the particulars shown are by way of example and for purpose of illustrative discussion of the preferred embodiments of the invention.

### Example 1

**Table 1-Pharmaceutical composition**

| **Sr. No.** | **Ingredients** | **% of ingredient by weight of core tablet** |
|---|---|---|
| 1 | Prasugrel base | 0.1-10% |
| 2 | Filler/ Diluents (e.g. MCC, Mannitol, Lactose,) | 40-85% |
| 3 | surfactant (e.g. Docusate sodium, poloxamers, polysorbate) | 0.1-10% |
| 4 | Disintegrant (e.g. CCS, SSG, Crospovidone) | 0-10% |
| 5 | Binder (e.g. HPC, HPMC , Povidone) | 0.1-10% |
| 6 | Stabilizer (Beta cyclodextrin) | 0 - 15% |
| 6 | Lubricant(e.g. SSF , sucrose stearate) | 0.1-5% |
| 7 | Optionally Film Coating | q.s |

### Preparation process:

a) Dissolving a Prasugrel base and surfactant in the acetone;
b) Dissolving binder in Acetone: Methanol mixture;
c) Mixing of the mixtures of step a) and b);
d) Granulating the mixture of step c) with inactive excipients including stabilizers by top spray granulation technology followed by drying;
e) Mixing the dried granules with one or more pharmaceutically acceptable excipients;
f) Compressing the blend to form the tablet and
g) Optionally coating the tablet.

### Example 2

**Table 2 - Pharmaceutical composition**

| **Sr. No.** | **Ingredients** | **% of ingredient by weight of core tablet** |
|---|---|---|
| 1 | Prasugrel Base | 0.1-10% |
| 2 | Docusate Sodium | 0.1-10% |
| 3 | Hydroxy Propyl Cellulose (HPC) | 0.1-10% |
| 4 | solvent | q.s |
| 5 | Mannitol | 40-85% |
| 6 | Micro Crystalline Cellulose | |
| 7 | Croscarmellose sodium | 0-10% |
| 8 | Magnesium stearate | 0.1-5% |
| 9 | Opadry II Brown | q.s |
| 10 | Purified Water | q.s |

### Preparation process:

a) Dissolving a Prasugrel base and Docusate sodium in the acetone;
b) Dissolving HPC in Acetone: Methanol mixture;
c) Mixing of the mixtures of step a) and b);
d) Granulating the mixture of step c) with inactive excipients by top spray granulation technology followed by drying;
e) Mixing the dried granules with one or more pharmaceutically acceptable excipients;
f) Compressing the blend to form the tablet and
g) Optionally coating the tablet.

### Example 3

**Table 3- Pharmaceutical composition**

| **Sr. No.** | **Ingredients** | **% of ingredient by weight of core tablet** |
|---|---|---|
| 1 | Prasugrel Base | 0.1-10% |
| 2 | Docusate Sodium | 0.1-10% |
| 3 | Hydroxy Propyl Cellulose (HPC) | 0.1-10% |
| 4 | solvent | q.s |
| 5 | Mannitol | 40-85% |
| 6 | Micro Crystalline Cellulose | |
| 7 | Beta Cyclodextrin | 0.1-15% |
| 8 | Croscarmellose sodium | 0-10% |
| 9 | Sodium stearyl fumarate | 0.1-5% |
| 10 | Sucrose Stearate | 0.1-5% |
| 11 | Opadry II Brown | q.s |
| 12 | Purified Water | q.s |

### Preparation process:

a) Dissolving a Prasugrel base and Docusate sodium in the acetone;
b) Dissolving HPC in Acetone: Methanol mixture;
c) Mixing of the mixtures of step a) and b);
d) Granulating the mixture of step c) with inactive excipients including Beta cyclodextrin by top spray granulation technology followed by drying;
e) Mixing the dried granules with one or more pharmaceutically acceptable excipients;
f) Compressing the blend to form the tablet and
g) Optionally coating the tablet.

### Example 4

**Table 4 - Pharmaceutical composition**

| **Sr. No.** | **Ingredients** | **% of ingredient by weight of core tablet** |
|---|---|---|
| 1 | Prasugrel Base | 0.1-10% |
| 2 | Poloxamer | 0.1-10% |
| 3 | Hydroxy Propyl Cellulose (HPC) | 0.1-10% |
| 4 | solvent | q.s |
| 5 | Mannitol | 40-85% |
| 6 | Micro Crystalline Cellulose | |
| 7 | Beta Cyclodextrin | 0.1-15% |
| 8 | Croscarmellose sodium | 0-10% |
| 9 | Sodium stearyl fumarate | 0.1-5% |
| 10 | Sucrose Stearate | 0.1-5% |
| 11 | Opadry II Brown | q.s |
| 12 | Purified Water | q.s |

### Preparation process:

a) Dissolving a Prasugrel base and Poloxamer in the acetone;
b) Dissolving HPC in Acetone: Methanol mixture;
c) Mixing of the mixtures of step a) and b);
d) Granulating the mixture of step c) with inactive excipients including (Beta) cyclodextrin by top spray granulation technology followed by drying;
e) Mixing the dried granules with one or more pharmaceutically acceptable excipients;
f) Compressing the blend to form the tablet and
g) Optionally coating the tablet.

### Example 5

The Comparative dissolution study was performed on prasugrel Base formulation with or without anionic surfactant.

**Table 5 - Comparative dissolution study**

| Dissolution Media | pH 4.0 Citrate phosphate, Paddle at 75 rpm | |
|---|---|---|
| Dissolution time points | Formulation Without surfactant | Formulation With anionic surfactant |
| 10 min | 68 | 99 |
| 20 min | 84 | 100 |
| 30 min | 89 | 98 |
| 45 min | 91 | 96 |

From the above data, it can be concluded that addition of anionic surfactant improves solubility of prasugrel base in release media.

### Example 6

The comparative bioequivalence study was performed on prasugrel base formulation prepared by Solid Dispersion Process and Wet granulation Process

**Table 6 - Comparative bioequivalence study**

| Process | Wet granulation Process | | | Solid Dispersion Process | | |
|---|---|---|---|---|---|---|
| Parameter | AUC(0-t) (h*ng/mL) | AUC(0-inf) (h*ng/mL) | Cmax (ng/mL) | AUC(0-t) (h*ng/mL) | AUC(0-inf) (h*ng/mL) | Cmax (ng/mL) |
| Geometric LSM Ratio (in %) Test / Reference | 93.83 | 95.00 | 96.94 | 100.86 | 101.24 | 92.56 |
| Intra-Subject Variability (CV %) | 16.84 | 16.72 | 35.56 | 15.17 | 13.79 | 19.13 |
| Power (%) | 90.56 | 90.87 | 40.35 | 97.53 | 98.91 | 88.84 |

### Example 7 -

The comparative stability study was performed on prasugrel base formulation with different packaging against EFIENT^{®} (Reference formulation)

**Table 7 -Stability data of EFIENT^{®} at 40°C/75% RH for 6 month**

| | | Initial | 3 Months | 6 Months |
|---|---|---|---|---|
| Related Substance (%) | Imp-A Peak 1 | 0.103 | 0.289 | 0.400 |
| | Imp-A Peak 2 | 0.171 | 0.374 | 0.472 |
| | Imp-B | 0.049 | 0.043 | 0.042 |
| | Imp-D | 0.012 | 0.012 | 0.011 |
| | Max. Unknown | 0.075 | 0.090 | 0.837 |
| | Total imp. | 0.668 | 1.207 | 2.266 |

**Table 8 - Stability data of Prasugrel base Formulation at 40°C/75% RH in Alu-Alu blister containing desiccant tablet**

| | | Initial | 3 Months | 6 Months |
|---|---|---|---|---|
| Related Substance (%) | Imp-A Peak 1 | 0.069 | 0.182 | 0.210 |
| | Imp-A Peak 2 | 0.073 | 0.190 | 0.222 |
| | Imp-B | 0.014 | 0.000 | 0.013 |
| | Imp-D | 0.033 | 0.028 | 0.032 |
| | Max. Unknown | 0.126 | 0.088 | 0.086 |
| | Total imp. | 0.653 | 0.791 | 0.972 |

**Table 9- Stability data of Formulation of Prasugrel base at 40°C/75% RH in Alu-Alu blister:**

| | | Initial | 3 Months | 6 Months |
|---|---|---|---|---|
| Related Substance (%) | Imp-A Peak 1 | 0.069 | 0.195 | 0.199 |
| | Imp-A Peak 2 | 0.073 | 0.201 | 0.213 |
| | Imp-B | 0.014 | 0.005 | 0.010 |
| | Imp-D | 0.033 | 0.033 | 0.028 |
| | Max. Unknown | 0.126 | 0.157 | 0.318 |
| | Total imp. | 0.653 | 0.947 | 1.234 |

## Claims

1. A Stable oral pharmaceutical composition comprising Prasugrel base, at least one surfactant and one or more pharmaceutically acceptable excipients.

2. The composition as claimed in claim 1,wherein the said composition comprising
(i) prasugrel base and (ii) one or more of the following excipients:
(a) 0.1 % to 10 % w/w at least one surfactant,
(b) 40 % to 85 % w/w at least one Filler,
(b) 0.1 % to 10 % w/w at least one Binder,
(d) 0.1 % to 10 % w/w at least one Disintegrant
(e) 0.1 % to 5 % at least one Lubricant

3. The composition as claimed in claim 2, wherein the said
Surfactant is selected from the group consisting of docusate sodium, poloxamer, sodium lauryl sulphate, polysorbate and combination thereof,
Filler is selected from the group consisting of microcrystalline cellulose, mannitol, lactose monohydrate and combination thereof.
Binder is selected from the group consisting of hydroxypropyl cellulose, hydroxypropyl methyl cellulose and povidone and combination thereof. Disintegrant is selected from the group consisting of croscarmellose sodium, sodium starch glycolate, and crospovidone and combination thereof.
Lubricant is selected from the group consisting of magnesium stearate, sodium stearyl fumarate and sucrose stearate and combination thereof.

4. The composition as claimed in claim 1, wherein the said
Surfactant is selected from the group consisting of docusate sodium, poloxamer, sodium lauryl sulphate, polysorbate and combination thereof,
and wherein said composition comprises one or more of, and optionally all of, the following excipients:
a filler selected from the group consisting of microcrystalline cellulose, mannitol, lactose monohydrate and combination thereof,
a binder selected from the group consisting of hydroxypropyl cellulose, hydroxypropyl methyl cellulose and povidone and combination thereof,
a disintegrant selected from the group consisting of croscarmellose sodium, sodium starch glycolate, and crospovidone and combination thereof,
a lubricant selected from the group consisting of magnesium stearate, sodium stearyl fumarate and sucrose stearate and combination thereof

5. The composition as claimed in any one of claims 1 to 4, wherein the said Prasugrel base in a micronized form having particle size
D10 is not more than 5.0 microns
D50 is Between 5.0 to 10.0 microns
D90 is not more than 20.0 microns.

6. The composition as claimed in any one of claims 1 to 5, wherein, the said composition is a granules, capsules, tablet and a film coated tablet.

7. The composition as claimed in any one of claims 1 to 5, wherein the said composition comprising micronized prasugrel base and at least one surfactant, is prepare by following process;
a. Dissolving a Prasugrel base and surfactant in the acetone;
b. Dissolving binder in Acetone: Methanol mixture;
c. Mixing of the mixtures of step a) and b);
d. Granulating the mixture of step c) with filler by top spray granulation technology followed by drying;
e. Mixing the dried granules with one or more pharmaceutically acceptable excipients like disintegrant, filler and lubricant.
f. Compressing the blend to form the tablet and
g. Optionally coating the tablet.

8. The composition as claimed in any one of claims 1 to 7, wherein the said composition comprising at least one surfactant and is stable after being stored at 40° C. and 75% relative humidity for the period of 6 months when pack in an aluminium blister-pack serrated with moisture absorbing channels with desiccant tablet in blister.

9. The composition as claimed in any one of claims 1 to 8, wherein the said composition contains not more than 1.5 % of total impurities when the prasugrel base formulation packed with blister containing desiccant tablet.

10. A process of preparing a composition as claimed in any one of claims 1 to 9 in the form of a tablet, which process comprises:
a. Dissolving a Prasugrel and a surfactant in acetone;
b. Dissolving a binder in Acetone: Methanol mixture;
c. Mixing of the mixtures of step a) and b);
d. Granulating the mixture of step c) with one or more pharmaceutically acceptable excipients by top spray granulation technology followed by drying;
e. Mixing the dried granules with one or more pharmaceutically acceptable excipients.
f. Compressing the blend (of the mixed dried granules with one or more pharmaceutically acceptable excipients) to form a tablet and
g. Optionally coating the tablet

11. A process according to claim 10, wherein in step d said excipients are inactive excipients, and preferably include one or more fillers.

12. A process according to claim 10 or 11, wherein in step d said excipients include one or more stabilizers.

13. A process according to any one of claims 10 to 12, wherein in step e said excipients include one or more of, and optionally all of, the following excipients: disintegrant, fillerand lubricant.

14. A composition as defined in any one of claims 1 to 9 for use in a method of treating a patient to reduce the rate of thrombotic cardiovascular events with acute coronary syndrome by administering said composition

15. A composition for use according to claim 14, wherein said patient is suffering from one or more of unstable angina, Non-ST-Elevation Myocardial Infraction (NSTEMI) and ST-Elevation Myocardial Infraction (STEMI),
and/or wherein said method comprises administering a unit dosage form of said composition.
